## Europäisches Patentamt
## European Patent Office
## Office européen des brevets

(11) Publication number: **0 227 240**
A1

## (12) EUROPEAN PATENT APPLICATION

(21) Application number: **86308044.6**

(22) Date of filing: **16.10.86**

(51) Int. Cl.⁴: **G 01 N 33/543**
G 01 N 33/68, G 01 N 33/72
//G01N33/577

(30) Priority: **16.10.85 GB 8525475**

(43) Date of publication of application:
**01.07.87 Bulletin 87/27**

(84) Designated Contracting States:
**AT BE CH DE ES FR GB GR IT LI LU NL SE**

(71) Applicant: **Farmos-Yhtymä Oy**
**P.O. Box 425**
**SF-20101 Turku 10(FI)**

(72) Inventor: **Hammond, Geoffrey Lewis**
**RR2 Lambeth**
**Ontario(CA)**

(74) Representative: **Collier, Jeremy Austin Grey**
**J.A.Kemp & Co. 14, South Square Gray's Inn**
**London WC1R 5EU(GB)**

(54) **Method for the immunoassay of a macromolecular analyte.**

(57) Neg-protein analytes can be assayed by a technique in which they are bound to immobilized boronic acid derivative adsorbents, and then detected using labeled antibodies as the probe. An important application of this method is that any neg-protein, separated by an affinity interaction with an immobilized support containing, for example, a dihydroxyboryl moiety, may be quantified directly and specifically using labeled antibodies. The technique permits rapid and specific determinations of the concentrations of non-enzymatically glycosylated forms of blood proteins that are important monitors of the control of diabetic treatment. The simplicity and rapidity of the method make it suitable for routine clinical diagnostic use.

EP 0 227 240 A1

# METHOD FOR THE IMMUNOASSAY OF A MACROMOLECULAR

## ANALYTE

This invention relates to affinity-immunometric assay methods for the identification and/or quantification of macromolecular analytes, and, in particular, neg-proteins.

The method of the present invention for the immunoassay of neg-protein analyte comprises contacting a sample containing said analyte with a boronic acid derivative, especially a phenyl-boronic acid, bound to a solid phase, contacting said solid phase with a labelled antibody to the said analyte, separating the solid phase with label bound thereto and then measuring the bound or the unbound label.

The new method may be used to determine the relative amounts of proteins, e.g. hemoglobin and albumin, that are present in blood samples in a non-enzymatically glycosylated (neg) form (a parameter which has been advocated as a convenient indicator for the diagnosis and management of diabetes mellitus. See I. Peacock, Journal of Clinical Pathology, 37: 841 (1984) and K. Miedema & T. Casparie, Annals of Clinical Biochemistry, 21: 2 (1984)).

Several methods are available for the clinical management of diabetic patients, but measurements of the blood levels of neg-proteins have been shown to correlate well with physicians' ratings of diabetic control, fasting blood glucose concentrations, mean and peak blood glucose concentrations, home urine test results, 24 hour glycosuria and home monitoring of blood glucose concentrations. In this regard, measurement of glycosylated hemoglobin $A_{1c}$ is particularly useful, because it is more plentiful than the other easily separable glycosylated hemoglobins and its concentration varies widely in relation to diabetic control. Glycosylated hemoglobin $A_{1c}$ is the most abundant of a group of four glycosylated derivatives of hemoglobin A (HbA), designated collectively as $HbA_1$, which account for approximately 6-9% of the total concentrations of hemoglobin in normal persons and as much as 20% in poorly controlled diabetics.

The non-enzymatic glycosylation of hemoglobin (Hb) occurs gradually during the 120 day life span of erythrocytes. Measurements of $HbA_1$ therefore reflect the average blood glucose values of the preceding weeks, and improvements in the control of diabetic patients result in a reduction in neg-Hb levels only after a 3-4 week period. The life span of plasma albumin is considerably shorter (20-30 days) than that of neg-Hb. Measurements of plasma neg-albumin levels may consequently reflect improvements in diabetic control within a one week period, and thereby provide a more rapid assessment of patient compliance. In contrast to the lag between improvements in diabetic control and changes in neg-protein levels, relatively short periods of poor control can rapidly and irreversibly increase neg-Hb and neg-albumin levels in the blood. For this reason, measurements of these parameters are a clinically valuable adjunct to urinary and blood glucose determinations, especially when patient compliance is poor, for example in young children, or when strict diabetic control is essential, as it is for instance during pregnancy.

Several methods are in widespread clinical use for the determination of neg-protein levels in blood samples, including isoelectric focusing, electrophoresis/-electroendosmosis, cation exchange chromatography, affinity chromatography, spectrometry and colorimetry. Many of these methods are specifically designed for the analysis of $HbA_{1c}$, about 75% of which is glycosylated, and cannot easily be modified for the analysis of other neg-proteins, such as neg-albumin. In addition, they are generally tedious, sensitive to minor variations in temperature, pH or ionic strength and, in some cases, require the use of highly specialized instruments, such as a scanning densitometer which must be carefully adjusted to ensure good between-batch assay precision. There is therefore a need for simple, accurate and precise routine assay that is generally applicable for the measurement of neg-forms of hemoglobin and albumin or any other clinically important protein. Affinity chromatography based on the use of phenylboronic acid appears to satisfy most of these criteria, and at least two companies (Amicon Corporation and Pierce Chemical Company) have developed kits for clinical use, and have published proposals for the use of immobilized phenylboronic acid for isolating and quantifying neg-proteins (see UK Patent Application GB 2024829 A, US Patent 4,269,605). These commercially available kits are, however, based on the use of conventional affinity column chromatographic procedures that require large amounts of expensive affinity gels, which may only be reused on a limited number of occasions without reduction in their efficiency. In comparison with many other methods, they are also labor intensive, require relatively large sample volumes, and rely on methods for the measurement of protein concentrations that are not entirely specific.

Immunoassay procedures are widely used to quantify specific component molecules in biological samples, in particular blood samples from patients for prognostic, diagnostic, or other clinical purposes. At present, the most important immunochemical analytical methods employ the use of so-called labels, which are moieties which are relatively easy to measure such as radioactive isotopes, molecules with fluorescent or bioluminescent properties, or enzymes which promote colour changes.

In conventional immunoassays, a pure preparation of analyte or an analogue of the analyte, is coupled to an appropriate label depending on the method of detection used.

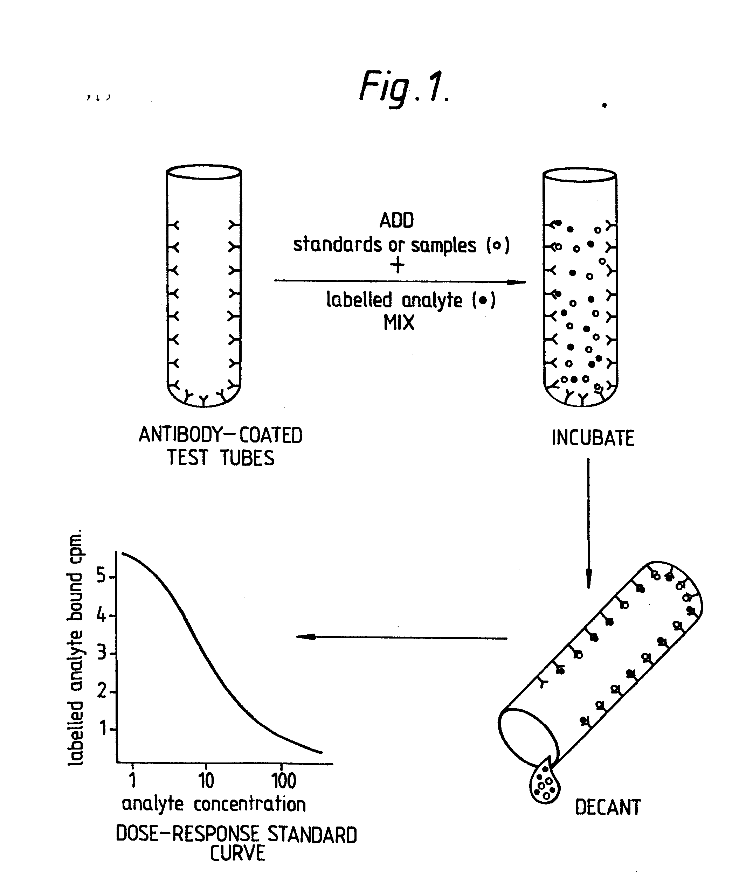

For example, in a radioimmunoassay a radioactive isotope is used as the label, which can be quantified in a liquid or crystal scintillation spectrometer. This labeled analyte, or analyte analogue, is incubated at a fixed concentration with a fixed amount of antibody that recognizes the labeled analyte as well as the analyte itself. Competition between labeled and unlabeled analyte for the antibody binding site therefore occurs and the amount of labeled analyte bound by the antibody is inversely proportional to the amount of unlabeled analyte present. Antibody-bound complexes are separated, for example, by immunoabsorption, physico-chemical adsorption or precipitation of either antibody-bound complexes or unbound analyte (labeled and unlabeled). Antibody-bound labeled analyte is then quantified and a standard curve is constructed from known analyte concentrations (standards), from which unknown concentrations of analyte may be interpolated. This is illustrated in the accompanying Figure 1.

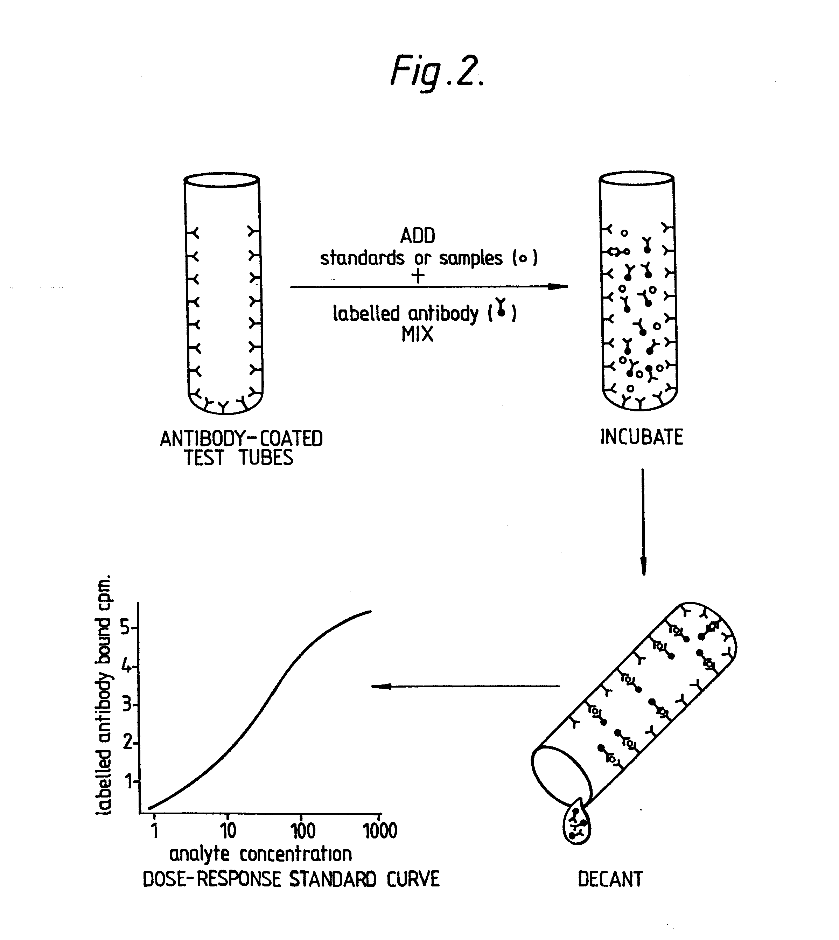

Immunometric assays, on the other hand, involve the use of labeled antibodies in place of labeled analyte and are particularly useful for measurements of large molecules, e.g. proteins, which possess two or more discrete antigenic sites, or epitopes. In this type of assay, illustrated in Figure 2, an antibody (or several antibodies) is used as "catching antibody", and this may be immobilized on a solid-phase support. The "labeled antibody" is a preparation of one or more antibodies which preferably recognize a different epitope(s) to the "catching antibody", although this is not essential. During incubation with standard amounts of analyte, or unknown concentrations of analyte in biological samples, a "sandwich" is formed between the "catching antibody", the analyte, and the "labeled antibody". Therefore in the presence of excess amounts of both "catching" and "labeled" antibodies, the amount of "labeled antibody" attached to such a "sandwich" is directly proportional to the amount of analyte present, and thereby provides the basis of a quantitative measurement. Immunometric techniques offer numerous advantages over conventional immunoassays, e.g., they obviate the need for pure preparations of labeled analyte; the use of excess reagents also reduces the necessity for precision pipetting of reagents, increase reaction rates, and thereby reduces incubation times.

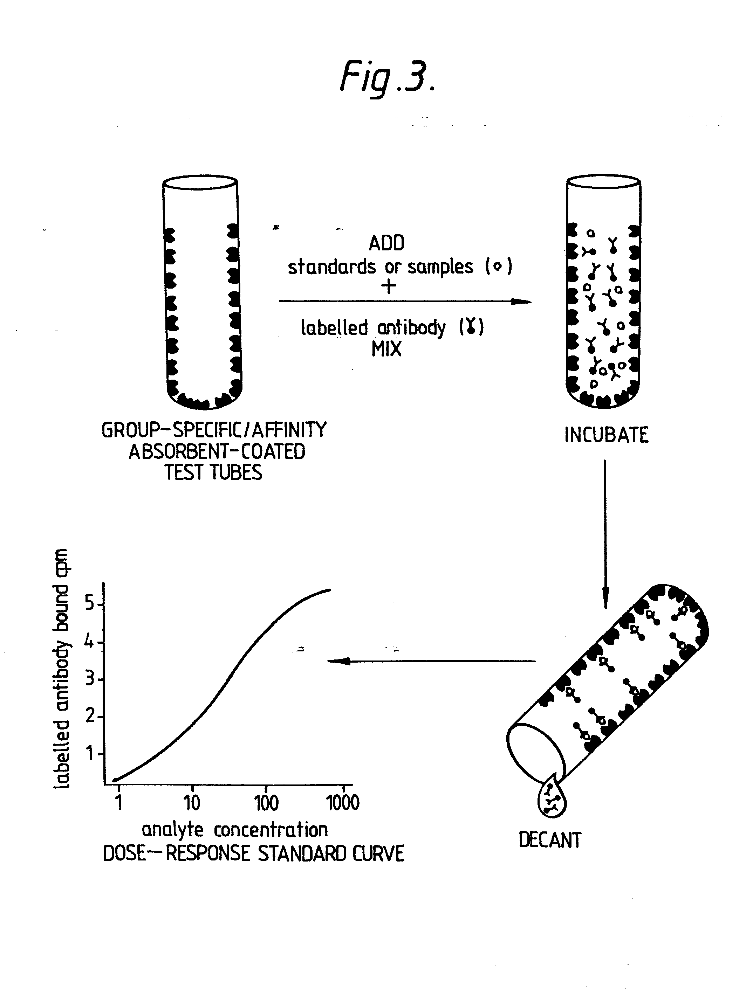

In the method of the present invention, labeled antibodies against specific neg-proteins are used in an excess reagent immunometric assay, in conjunction with an immobilized, boronic acid derivative adsorbent instead of the conventional immobilized "catching antibody", to quantify the concentration of the neg-protein in a biological sample. The method is sensitive, specific, accurate and precise, and because of its simplicity it is suitable for routine analyses of large numbers of samples. The general principle of the method, illustrated in Figure 3, can be applied to the detection and determination of a variety of neg-protein analytes that may be isolated on the basis of a high affinity interaction with an immobilized boronic acid derivative adsorbent.

The neg-proteins can be isolated by an affinity interaction with an immobilized boronic acid derivative adsorbent, which may be attached to an insoluble matrix, such as; polyacrylamide, dextran, beaded agarose, a cellulose derivative, clay (kaolin), glass, nylon (i.e. a polyamide), a polyester, polystyrene, a polyolefin, a polyvinylidene halide, a metal oxide, a porous ceramic, diatomaceous earth, or magnetic particles or any other suitable material. The major advantage of using an immobilized boronic acid derivative matrix is that it will reversibly bind neg-proteins with relatively high affinity, and thereby enable contaminating substances to be physically washed away by the use of centrifugation and aspiration of supernatants, or by filtration. The boronic acid derivative adsorbent may also be coated onto tubes, cuvettes, magnetic particles or dipsticks, in order to facilitate the washing process. The adsorbed neg-protein may then be removed from the matrix if necessary, by physical alteration of the binding conditions (e.g. pH), or desorption with a competitive binding ligand or denaturing agent.

Any immobilized boronic acid derivative adsorbent may be substituted for the "catching antibody" used in conventional immunometric assays, for example a phenyl-boronic acid. The advantages that this affords are numerous; for instance, a) it obviates the need for a constant supply of large amounts of specific antibodies required for immobilization, b) the affinity interaction between boronic acid derivative adsorbents and neg-proteins is almost instantaneous when compared to the relatively slow reaction between antibody and antigen, and c) recovery of the adsorbed neg-protein by desorption is obviated because measurements are accomplished in situ by the use of labelled antibodies.

The affinity-immunometric assay method of the invention represents an important addition to the current repertoire of clinical chemical and microbiological laboratory tests. In particular, affinity-immunometric assays can be used to measure the plasma concentrations of neg-proteins whose biological activity may be related in some way to their carbohydrate moieties, and may be especially useful in determinations of blood neg-protein levels for the clinical management of diabetic patients. Assay protocols that illustrate these applications, and which are suitable for use in commercial kit form, are presented in the following examples:

## EXAMPLE 1

This example demonstrates the use of immobilized phenylboronic acid in an affinity-immunometric assay for neg- hemoglobin.

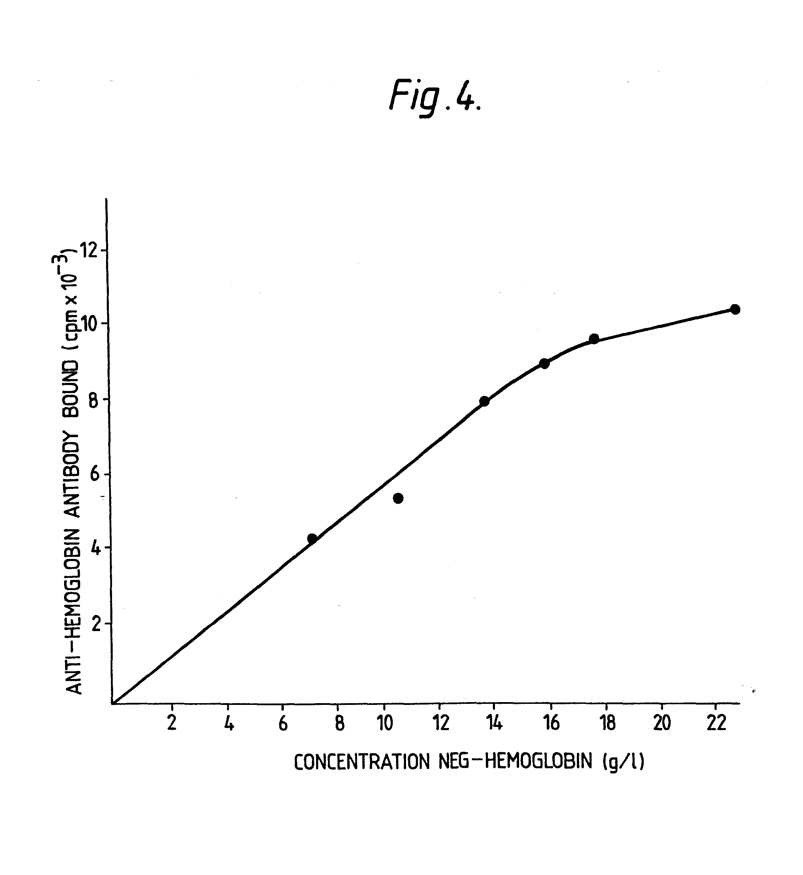

Blood samples and standards were prepared similarly, i.e. 50 µl aliquots were added to 50 µl of lysing solution, containing 1% saponin and EDTA. The tubes were vortex mixed and incubated for 5 min prior to the addition of 900 µl of assay buffer (0.1 M glycine, 0.01 M $MgCl_2$, 0.1% $NaN_3$, pH 8.3). The tubes were again vortex mixed and 100 µl samples were taken in duplicate and added to tubes containing 100 µl of a  slurry of phenylboronic acid – Sepharose affinity gel. This mixture was gently agitated for 10 min, 2 ml of assay buffer was added to each tube and these were then centrifuged at 1,000 G for 5 min.  The supernatants were aspirated, and the gel pellets were again washed with 2 ml assay buffer in the same way.  The $^{125}$I-labeled hemoglobin antibody (~40,000 cpm / 200 µl in assay buffer containing 5% fetal calf serum) was added to all tubes and these were then incubated for 1 hr with intermittent shaking.  Assay buffer (2 ml) was added to all tubes and they were then centrifuged (1,000 G for 5 min) and the supernatants were aspirated to waste.  This was repeated and the gel pellets were counted for 1 min in a gamma counter.  The concentration of neg-Hb in the samples was interpolated from a standard curve (Figure 4) generated using controls in which the concentration of neg-Hb was known, and the total Hb concentrations were measured spectrophotometrically (430 nm) in the same samples after dilution (1:40) in assay buffer.

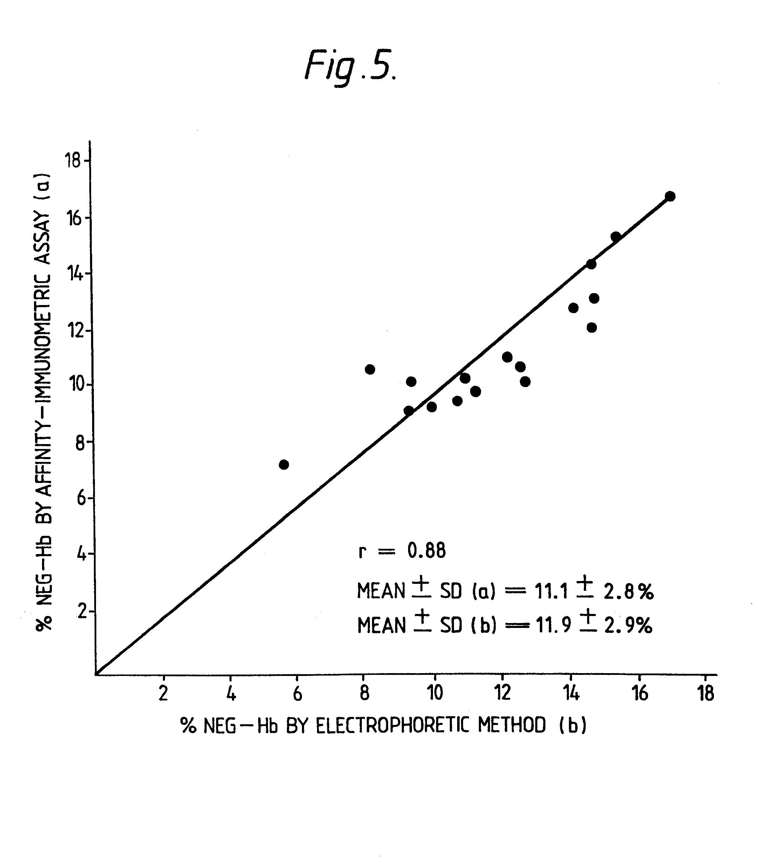

A comparison (Figure 5) was also made between the percentages of neg-Hb measured in blood samples by the affinity-immunometric assay and commercially available electrophoretic method.

### EXAMPLE 2

This example demonstrates the use of immobilized phenylboronic acid in an affinity-immunometric assay for neg-albumin.

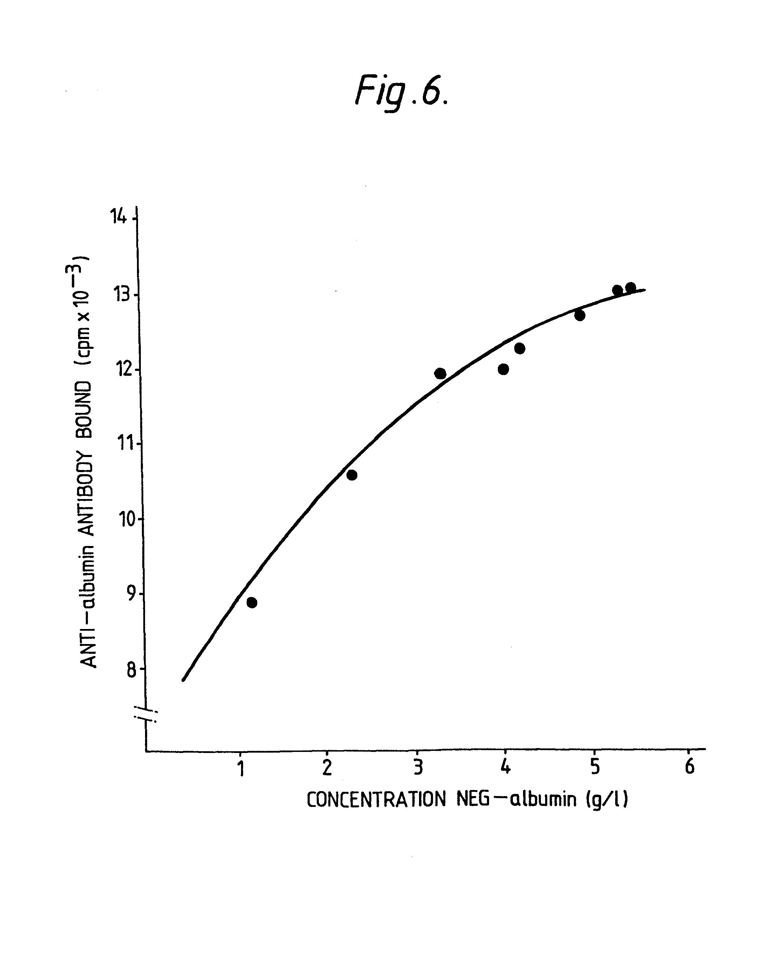

Blood samples and standards may be prepared as described in Example 1 or, alternatively, blood plasma or serum may be diluted 1:20 in the same assay buffer used in Example 1. Aliquots (100 µl) of the diluted lysed blood or plasma samples are added to tubes containing 100 µl of an immobilized slurry of phenylboronic acid - Sepharose affinity gel. This mixture is gently agitated for 10 min, 2 ml of assay buffer is added to each tube and these are then centrifuged at 1,000 g for 5 min. The supernatants are aspirated, and the gel pellets are again washed with 2 ml assay buffer in the same way. The $^{125}$I-labeled albumin antibody (~40,000 cpm / 200 µl assay buffer containing 5% fetal calf serum) is then added to all tubes which are incubated for 1 hr with intermittent shaking. Assay buffer (2 ml) is added to all tubes, and after centrifugation (1,000 g for 5 min) the supernatants are aspirated. This is repeated and the gel pellets are counted for 1 min in a gamma counter. The concentration of neg-albumin may be interpolated from a standard curve (Figure 6) generated using plasma samples in which the concentration of neg-albumin was determined using a commercially available boronate affinity chromatography kit (Pierce GlycoTest$^{TM}$). These data also serve to demonstrate the good correlation that exists between the values obtained by the affinity-immunometric assay and a standard affinity chromatographic method.

In the above examples the affinity ligand has been attached to Sepharose as the solid-phase support. This is an efficient and convenient immobilization matrix, but it suffers from the disadvantage that the non-specific trapping of labeled antibodies is a significant problem. This may be overcome by using an alternative solid-phase support that is associated with very low non-specific adsorption, such as controlled pore glass or kaolin.

## CLAIMS

1. Method for the immunoassay of a neg-protein analyte, which comprises contacting a sample containing said analyte with a boronic acid derivative adsorbent bound to a solid-phase, contacting said solid-phase with a labelled antibody to the said analyte, separating the solid-phase with label bound thereto and then measuring the bound or the unbound label.

2. Method according to claim 1 in which the said solid phase comprises polyacrylamide, dextran, beaded agarose, a cellulose derivative, a polyamide, a polyester, polystyrene, a polyolefin, a polyvinylidene halide, a metal oxide, a porous ceramic, kaolin, diatomaceous earth, glass, or magnetic particles.

3. Method according to claim 1 or 2 in which the said labelled antibody is a polyclonal or monoclonal antibody raised against the said neg-protein analyte and labelled with a radioisotope, an enzyme, or a fluorescent, luminescent, phosphorescent or spin-labelled molecule.

4. Method according to any one of the preceding claims wherein the boronic acid derivative is a phenyl-boronic acid.

5.  An assay kit for use in the method of any of
the preceding claims comprising: (1) a labeled antibody
to said neg-protein analyte; (2) a boronic acid derivative
adsorbent bound to a solid phase; and (3) calibration
standards of the neg-protein analyte.

6.  An assay kit according to claim 5 wherein the
boronic acid derivative adsorbent is bound indirectly to
the solid phase by a second binding agent.

# Fig.1.

ADD
standards or samples (o)
+
labelled analyte (•)
MIX

ANTIBODY—COATED
TEST TUBES

INCUBATE

DECANT

labelled analyte bound cpm.

5

4

3

2

1

1        10        100

analyte concentration

DOSE-RESPONSE STANDARD
CURVE

# Fig.2.

ADD
standards or samples (o)
+
labelled antibody (Y)
MIX

ANTIBODY–COATED
TEST TUBES

INCUBATE

labelled antibody bound cpm.

analyte concentration

DOSE-RESPONSE STANDARD CURVE

DECANT

3/6

0227240

Fig.3.

GROUP-SPECIFIC/AFFINITY
ABSORBENT-COATED
TEST TUBES

ADD
standards or samples (o)
+
labelled antibody (Y)
MIX

INCUBATE

DECANT

labelled antibody bound cpm

analyte concentration
DOSE–RESPONSE STANDARD CURVE

# Fig.4.

Fig.5.

# Fig.6.

## EUROPEAN SEARCH REPORT

European Patent
Office

### DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y,D | US-A-4 269 605  (P.D.G. DEAN et al.)<br>* columns 1-3 * | 1-6 | G 01 N  33/543<br>G 01 N  33/68<br>G 01 N  33/72 //<br>G 01 N  33/577 |
| Y | EP-A-0 111 211  (MILES LABORATORIES, INC.)<br>* page 5, paragraph 2; pages 25-28, example 2; claims 1-8 * | 1-6 | |
| A | US-A-4 478 744  (L.M. MEZEI et al.)<br>* column 2, lines 13-68 * | 1 | |
| A | EP-A-0 063 064  (LABORATOIRE DES STALLERGENES) | | |
| | ----- | | **TECHNICAL FIELDS SEARCHED (Int. Cl.4)**<br><br>G 01 N  33/00 |

The present search report has been drawn up for all claims

| Place of search | Date of completion of the search | Examiner |
|---|---|---|
| BERLIN | 20-02-1987 | GREEN C.H. |